# EUROPEAN PATENT SPECIFICATION

(11) **EP 3 492 006 B1**
(45) Date of publication and mention of the grant of the patent: **14.05.2025**
(21) Application number: 17205016.3
(22) Date of filing: 01.12.2017
(51) Int. Cl.: A61B 5/259

(54) **METHODS AND APPARATUS FOR PROVIDING ELECTRICAL CONNECTION TO A SUBJECT**
VERFAHREN UND VORRICHTUNG ZUR BEREITSTELLUNG EINER ELEKTRISCHEN VERBINDUNG FÜR EINE PERSON
PROCÉDÉ ET APPAREIL DE FOURNITURE DE CONNEXION ÉLECTRIQUE À UN SUJET

(43) Date of publication of application: 05.06.2019
(73) Proprietor: Nokia Technologies Oy, 02610 Espoo (FI)
(72) Inventor: Radivojevic, Zoran, Cambridge, Cambridgeshire CB3 0FA (GB); Bruna, Matteo, Cambridge, Cambridgeshire CB3 0FA (GB); Zarra, Salvatore, Cambridge, Cambridgeshire CB3 0FA (GB); Sassi, Ugo, Cambridge, Cambridgeshire CB3 0FA (GB)
(74) Representative: Swindell & Pearson Limited

(56) References cited:
- EP-A2- 0 217 383
- WO-A1-2016/009277
- WO-A2-2008/017921
- JP-U- H0 515 907
- US-A- 4 067 342
- US-A1- 2009 105 574
- US-A1- 2016 089 045
- US-A1- 2017 258 357
- US-B1- 9 827 430

## Description

### TECHNOLOGICAL FIELD

Examples of the disclosure relate to methods and apparatus for providing electrical connection to a subject. In particular, they relate to methods and apparatus for providing electrical connection to a subject using a wearable electronic device.

### BACKGROUND

Apparatus for detecting bio-parameters are known. These may require electronic connections between one or more electrodes and the skin of a subject. It is useful to provide methods and apparatus for detecting such bio-parameters which can be implemented using a wearable electronic device.

EP 0 217 383 discloses an electrocardiographic electrode which is held in close contact with a user's skin in order to detect a biopotential signal from the user. The electrode comprises a viscous base member which is viscous on the back side to be held in contact with the skin and on the front side to be coupled to a lead connector.

WO 2008/017921 discloses a system for detecting bioelectric signals from a user. A series of detection electrodes are held in place on the skin to detect bioelectric signals. The detection electrodes are magnetically coupled to removable connection elements.

JPH0515907 discloses an electrode for low-cost manufacture that may be easily attached to and detached from a patient.

US 4067342 discloses a tape electrode for the transmission of electrical signals into the human body through the skin. One side of the tape is a conductive material with an adhesive for adhesion to the user's skin. The other side of the tape is connected to a lead wire by magnetic attraction. Other relevant prior art is provided in US 2009/0105574 A1, US 2017/0258357 A1, WO 2016/009277 A1, US 2016/0089045 A1, US 9 827 430 B1.

### BRIEF SUMMARY

The invention is defined in the independent claims. According to various, but not necessarily all, examples of the disclosure there is provided a method comprising: applying a conductive contact to a subject's skin; positioning an electrode relative to the conductive contact to enable an electrical connection between the subject's skin and the electrode wherein the electrical connection enables a bio-parameter of the subject to be detected; and wherein at least one of the conductive contact and the electrode are magnetised so as to enable a magnetic field from the magnetised at least one conductive contact and/or electrode to align the conductive contact and the electrode.

Both the conductive contact and the electrode may be magnetised.

The electrode may be provided within a wearable electronic device.

The electrode may comprise a conductive film mounted on a textile substrate

The magnetic field may be arranged to hold the electrode in position overlaying the conductive contact.

The magnetic field may be arranged to pull the electrode back to a position overlaying the conductive contact if the electrode is moved relative to the conductive portion.

The conductive contact may comprise an adhesive and a conductive film overlaying the adhesive wherein the adhesive is applied to the surface of the subject's skin. The adhesive may comprise magnetic particles suspended within the adhesive.

The conductive contact may comprise an ink comprising conductive particles which is deposited on the surface of the subject's skin.

The conductive contact may comprise an ink comprising conductive particles which is dispersed underneath the epidermis of the subject's skin.

The electrical connection may be a galvanic connection.

The electrical connection may comprise capacitive coupling between the conductive contact and the electrode.

The bio-parameter may comprise at least one of; a biopotential, a bio-mechanical output, an optical output.

According to various, but not necessarily all, examples of the disclosure there may be provided an apparatus comprising: at least one electrode wherein the at least one electrode is arranged to be coupled to a conductive contact on a subject's skin so as to enable an electrical connection between the conductive contact and the electrode wherein the electrical connection enables a bio-parameter of the subject to be detected; wherein at least one of the conductive contact and the electrode are magnetised so as to enable a magnetic field to align the conductive contact and the electrode.

The electrode may be magnetised.

The electrode may comprise a magnetic portion.

The electrode may comprise a conductive film mounted on a textile substrate.

The apparatus may comprise a plurality of electrodes.

The bio-parameter may comprise at least one of; a biopotential, a bio-mechanical output, an optical output.

According to various, but not necessarily all, examples of the disclosure there may be provided an adhesive arrangement comprising: a conductive contact overlaying an adhesive wherein the adhesive is arranged to be applied to the surface of a subject's skin so as to enable an electrical connection between the subject's skin and an electrode.

The adhesive arrangement may comprise magnetic particles suspended within the adhesive.

The adhesive may be is provided on a first substrate and arranged to be transferred from the first substrate to the subject's skin.

The first substrate may comprise paper.

According to various, but not necessarily all, examples of the disclosure there may be provided an ink comprising: conductive particles and arranged to be deposited on the skin of a subject so as to enable an electrical connection between the subject's skin and an electrode.

The ink may be arranged to be deposited on the surface of the subject's skin.

The ink may be arranged to be dispersed underneath the epidermis of the subject's skin.

The ink may comprise ferromagnetic particles.

According to various, but not necessarily all, examples of the disclosure there is provided examples as claimed in the appended claims.

### BRIEF DESCRIPTION

For a better understanding of various examples that are useful for understanding the detailed description, reference will now be made by way of example only to the accompanying drawings in which:
Fig.1 illustrates an examples apparatus;
Fig. 2 illustrates an example apparatus in use;
Fig. 3 illustrates another example apparatus in use; and
Fig. 4 illustrates an example method.

### DETAILED DESCRIPTION

Examples of the disclosure relate to methods and apparatus 1 providing electrical connection to a subject. The electrical connection could be used for detecting and measuring bio-parameters. The bio-parameters could comprise biopotentials, biomechanical outputs, optical outputs or any other suitable bio-parameters. In examples of the disclosure a conductive contact 23, 33, 35 can be attached to the skin 21 of a subject to provide an electrical connection between the subject's skin 21 and an electrode 3. One or both of the conductive contact 23, 33, 35 and the electrode 3 may be magnetized so as to provide a magnetic field which helps to secure the conductive contact 23, 33, 35 and the electrode 3 in position so as to maintain the electrical connection. This enables the bio-parameter to be reliably detected and measured. Examples of the disclosure also provide for improved comfort of the sensing apparatus 1 as reliable contacts to the subject's skin 21 can be made without using any tensioning devices such as rubber bands or tapes, which may be uncomfortable.

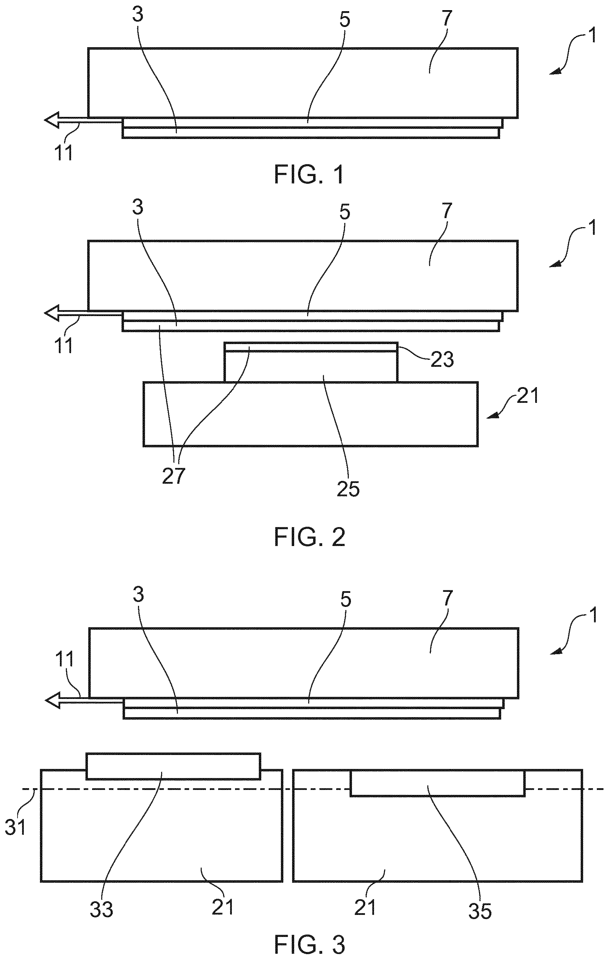

Fig. 1 schematically illustrates an example apparatus 1 according to examples of the disclosure. The apparatus 1 comprises at least one electrode 3, a magnetic portion 5 and a substrate 7.

The electrode 3 is mounted on the substrate 7. The electrode 3 comprises an electrically conductive material. The electrode 3 may comprise nickel, ferrite, ferrite oxides, copper, silver, carbon or any other suitable conductive material.

The electrode 3 may be flexible. The electrode 3 may be flexible so that it may be deformed while the apparatus 1 is in use. In some examples the electrode 3 may be flexible to enable the electrode to be deformed to match the surface of the subject's skin. The electrode 3 may be flexible so that it can be integrated into the fabric of an item of clothing which can then be worn by a subject. In other examples the electrode 3 may be flexible so that it can be integrated into a strap or other means that could be attached to the subject.

In some examples of the disclosure the electrode 3 may comprise a conductive film or conductive trace. The conductive film or conductive trace may be printed onto the substrate 7. The conductive film or conductive trace may be printed onto the substrate 7 in any suitable pattern.

The electrode 3 is arranged to be coupled to a conductive contact 23, 33, 35 which may be provided on a subject's skin 21. The electrode 3 may be coupled to the conductive contact 23, 33, 35 by being positioned overlaying the electrode 3. In some examples the electrode 3 may be provided on an outer surface of the apparatus 1 so that the electrode 3 can be arranged in direct contact with the conductive contact 23, 33, 35. This may provide for a galvanic connection between the conductive contact 23, 33, 35 and the electrode 3. The galvanic contact may provide a direct current path between the conductive contact 23, 33, 35 and the electrode 3.

In other examples the electrode 3 may be arranged to capacitively couple to the conductive contact 23, 33, 35. In such examples an insulating layer may be provided over the electrode 3 and/or conductive contacts 23, 33, 35 so that, in use, the insulating layer is positioned between the electrode 3 and the conductive contact 23, 33, 35. The insulating layer might prevent there being a galvanic connection between the electrode 3 and the conductive contact 23, 33, 35.

In the example apparatus of Fig, 1 the electrode 3 is magnetized. The magnetization of the electrode 3 provides a magnetic field which acts to attract the conductive contact 23, 33, 35 to the electrode 3. The magnetic field may be strong enough to retain the electrode 3 in position relative to the conductive contact 23, 33, 35. The magnetic field may be strong enough to move the electrode 3 towards the conductive contact 23, 33, 35 if the electrode 3 is positioned in close proximity to the conductive contact 23, 33, 35. In some examples the magnetic field may act to realign the electrode 3 and the conductive contact 23, 33, 35 if the electrode 3 is moved relative to the conductive contact 23, 33, 35. For instance, if the electrode 3 is provided in a wearable device then the wearable device and the electrode 3 may move as the subject moves which may cause displacement of the electrode 3 relative to the conductive contact 23, 33, 35. In such cases the magnetic field acts to realign the electrode 3 and the conductive contact 23, 33, 35 and restore them to their original configuration.

Any suitable means may be used to magnetize the electrode 3. In the example of Fig. 1 the apparatus 1 comprises a magnetized portion 5 which is provided underneath the conductive film. In other examples the material within the conductive trace may be magnetized.

The magnetized portion 5 may comprise any suitable magnetic material. In the example apparatus 1 of Fig. 1 the magnetized portion comprises magnetic paint. The magnetic paint may be deposited on the substrate 7 to provide a magnetic layer between the electrode 3 and the substrate 7. The magnetic paint may comprise magnetic rare earth metals such as neodymium, alloys comprising rare earth metals or any other suitable magnetic material in the form of inks or powders.

The magnetized portion 5 may be provided in a thin layer so that the magnetized portion 5 is flexible enough to be provided within a wearable device.

The apparatus 1 also comprise a substrate 7. The substrate 7 may be flexible and/or deformable. The substrate 7 may be flexible and/or deformable to enable the apparatus 1 to be worn by a subject. In some examples the substrate 7 may comprise a textile. This may enable the apparatus 1 to be integrated into an item of clothing which can be worn by the subject. In other examples the substrate 7 may form part of a strap which may be secured around a limb or torso of the subject. In such examples the substrate 7 may be flexible to enable the strap to be in close contact with the subject's skin 21.

In the example of Fig. 1 only one, electrode 3 is shown. It is to be appreciated that the apparatus 1 may comprise a plurality of electrodes 3 and associated magnetic portions 5. The plurality of electrodes 3 may be mounted on the same substrate 7. The number and positioning of the electrodes 3 may depend on the bio-parameter that is to be measured, the type of wearable device that the apparatus 1 is to be integrated into and/or any other suitable factors.

The electrode 3 is arranged to provide an output signal 11. The output signal 11 may be indicative of a detected bio-parameter. The detected bio-parameter may comprise any time varying electrical signal that is generated by the body of the subject. The bio-parameter signal may comprise an autonomic signal. The autonomic signal may be controlled subconsciously by the subject.

In some examples the bio-parameter may comprise a biopotential. The biopotential may comprise electrical signals that are generated within the subject's body by the subject's heartbeat. In some examples the biopotentials could comprise electrical activity of the subject's brain or other parts of their nervous system. The biopotential signal could comprise at least one of an electrocardiogram signal, electroencephalogram signal, electromyogram signal, electrooculogram signal, electrogastrogram signal, galvanic skin potential or any other suitable biopotential.

In some examples the bio-parameter may comprise a biomechanical biomechanical output. The biomechanical output may comprise mechanical signals that are generated within the subject's body by the subject's heartbeat, respiration, abdominal sounds or other body movements. In some examples the biomechanical output could comprise at least one of ballistocardiogram signal, seismocardiogram signal, phonocardiogram signal or any other suitable signal.

In some examples the bio-parameter may comprise an optical output. For example the bio-parameter could comprise a change in the optical properties of a part of the subject. The optical properties could be the absorbance or the reflectivity of part of the subject which may be time varying due to the subject's pulse, the subject's oxygenation levels or any other suitable factor.

It is to be appreciated that other bio-parameters could be used in other implementations of the disclosure.

Different apparatus 1 may be arranged to measure different bio-parameters. In some examples the same apparatus 1 may be arranged to measure more than one type of bio-parameter. The type of electrodes 3 and conductive contacts 23, 33, 35 that are used may be dependent upon the type of bio-parameter that is to be detected.

The output signal 11 may be provided to processing circuitry to enable the detected biopotential signals to be analysed and/or measured. In some examples the processing circuitry could be part of the wearable device so that the electrode 3 may be coupled directly to the processing circuitry. In other examples the processing circuitry could be provided in a different device. In such examples the wearable device may comprise one or more transceivers which may enable the information obtained by the electrode 3 to be transmitted to the processing circuitry.

Fig. 2 illustrates an example apparatus 1 in use. The example apparatus 1 comprises a substrate 7, a magnetic portion 5 and an electrode 3 which may be as described above.

In use the apparatus 1 is positioned adjacent to the skin 21 of a subject. The subject may be a person or an animal which generate bio-parameters which can be measured by the electrode 3. In some examples the apparatus 1 may be provided within a wearable electronic device which is worn by the subject. In other examples the apparatus 1 could comprise an electronic device or an electronic chip which is connected to the subject.

When the apparatus 1 is in use the apparatus 1 is positioned so that the electrode 3 is overlying a conductive contact 23 provided on a subject's skin 21. The electrode 3 may be positioned overlaying the conductive contact 23 so as to enable an electrical connection between the subject's skin 21 and the electrode 3. The electrical connection could be galvanic connection. In some examples the electrical connection could comprise capacitive coupling between the conductive contact 23 and the electrode 3.

The conductive contact 23 may comprise any suitable conductive material 27 which may enable an electrical connection to be provided between the skin 21 of the subject and the electrode 3. In some examples the conductive contact 23 and the electrode 3 may be formed from the same conductive material 27. In other examples different materials could be used. The conductive contact 23 may be formed from carbon black, gold, silver copper or any other suitable material.

In some examples the conductive contact 23 may be provided as a flexible film. The flexible film may allow the subject to move even when the conductive contact 23 is attached to their skin 21.

In some examples the conductive contact 23 may comprise a magnetic material. This may enable the conductive contact 23 to be magnetically attracted to the magnetic portion 5 of the apparatus 1.

The conductive contact 23 may be sized and shaped so that when the apparatus 1 is positioned overlaying the subject there is an overlap between the electrode 3 and the conductive contact 23. The overlap may ensure that the electrode 3 can be electrically coupled to the conductive contact 23.

In examples of the disclosure the conductive contact 23 is applied to the skin 21 of the subject. The application of the conductive contact 23 enables galvanic connection between the skin 21 of the subject and the conductive contact 23. This may provide a direct current path between the skin 21 of the subject and the conductive contact 23.

In the example arrangement of Fig. 2 the conductive contact 23 is applied to the subject's skin 21 by an adhesive 25. The adhesive 25 may comprise a gel such as hydrogel or any other suitable adhesive. The adhesive 25 is arranged so that the conductive contact 23 is provided overlaying the adhesive 25. This ensures that the conductive contact 23 is securely attached to the subject's skin 21.

The adhesive 25 may be a temporary adhesive. The temporary adhesive may be washed of the subject's skin 21 after the biopotential has been measured. This may enable the conductive contact 23 to be positioned on the subject's skin 21 for a short period of time. The short period of time may be several minutes or hours. The short period of time may be a time period sufficient to enable a biopotential to be measured.

In some examples the adhesive 25 may be conductive. This may provide a good electrical connection between the conductive contact 23 and the subject's skin 21.

In some examples the adhesive 25 may be magnetized. In such examples the adhesive 25 may comprise magnetic particles dispersed within the adhesive 25. The magnetic particles may comprise ferromagnetic particles including but not limited to metal oxides such as Fe₃O₄ and γ-Fe₂O₃, metals such as cobalt, nickel, iron and alloys of these with other elements as well as rare-earth compounds or any other suitable particles. In some examples the magnetic particles may be coated with polymers. Any suitable polyemrs may be used to coat the magnetic particles such as polyethylene imine, polylactic acid, silica-based matrices, polystyrene, chitosan, dextran or any other suitable polymer. The magnetic particles may be arranged to attract the electrode 3 in the apparatus 1.

In the example of Fig. 2 the apparatus 1 comprises a magnetic portion 5 and the adhesive 25 may comprise magnetic particles. This enables both the electrode 3 and the conductive contact 23 to be magnetized. In other examples only one of the electrode 3 and the conductive contact 23 might be magnetized. For instance, in some examples the electrode 3 might comprise a magnetic portion 5 but the conductive contact 23 is not magnetized while in other examples the conductive contact 23 could comprises magnetic particles but the electrode 3 is not magnetized.

In the example of Fig. 2 only one conductive contact 23 is shown. It is to be appreciated that a plurality of conductive contacts 23 may be positioned at different locations on the subject's skin 21. The position of the conductive contacts 23 may be dependent upon the bio-parameter that is to be measured, the type of subject that is being monitored or any other suitable factor. The plurality of conductive contacts 23 may be arranged in positions corresponding to the positions of a plurality of electrodes 3 within a wearable electronic device. This enables a plurality of electrodes 3 to be positioned overlaying the plurality of conductive contacts 23 and enables bio-parameters to be detected from different positions on the subject.

In the example of Fig. 2 the conductive contact 23 is temporarily attached to the subject's skin 2. Fig. 3 illustrates another example apparatus 1 in use. In the example of Fig. 3 conductive contacts 33, 35 that are more permanently attached to the subject's skin 21 are shown.

The example of Fig. 3 shows a semi-permanent conductive contact 33 and a permanent conductive contact 35. In these examples the conductive contacts 33, 35 are applied to the subject's skin 21 without any intervening materials between the conductive contact 33, 35 and the skin 21. There is no adhesive material provided between the conductive contacts 33, 35 and the subject's skin 21 in the examples of Fig. 3. The conductive contacts 33, 35 in the example of Fig. 3 may provide for reusable conductive contacts 33, 35.

The semi-permanent conductive contact 33 comprises a conductive material which is dispersed on the subject's skin 21. In the example of Fig. 3 the semi-permanent conductive contact 33 comprises a conductive ink. The conductive ink may comprise any conductive material such as nickel, ferrite, silver, gold, carbon or any other suitable, non-toxic, material. In some examples the conductive material may be magnetic so as to enable the conductive contact 33 to be attracted to a magnetic portion 5 of an electrode 3.

In some examples the semi-permanent conductive contact 33 may be magnetized. In such examples the conductive ink may comprise magnetic particles which may be dispersed within the conductive ink. The magnetic particles may comprise ferromagnetic particles such as iron oxide or any other suitable materials.

In the example of Fig. 3 the semi-permanent conductive contact 33 is deposited on the surface of the subject's skin 21. That is the semi-permanent conductive contact 33 does not go beneath the epidermis 31 of the subject's skin 21. In some examples the ink that forms the semi-permanent conductive contact 33 may be absorbed by the very top layer of the subject's skin 21 but does not get absorbed beyond the epidermis 31.

The conductive ink may remain on the subject's skin 21 for several days or weeks. The conductive ink might not be easily washed off the skin 21 to ensure that the semi-permanent conductive contact 33 remains on the subject.

Having the semi-permanent conductive contact 33 dispersed on the surface of the subject's skin 21 may provide for an improved electrical connection between the subject's skin 21 and the semi-permanent conductive contact 33 compared to the examples of Fig. 2.

The semi-permanent conductive contact 33 may be useful for circumstances where a bio-parameter is to be measured over a longer period of time such as several days or weeks. This may be useful for health monitoring where a subject may need to be checked at regular intervals over several days or weeks. This could be useful for fitness applications where a subject may be monitoring bio-parameters as they exercise over several days or weeks. In such examples, having the semi-permanent conductive contact 33 attached to the subject's skin 21 ensures that the electrodes 3 are always positioned on the same position on the subject. This ensures that consistent measurements are obtained by the electrodes 3 as it avoids variability caused by the electrodes 3 being positioned at different places.

The permanent conductive contact 35 comprises a conductive material which is dispersed, at least in part, underneath the epidermis 31 of the subject's skin 21. To ensure that the conductive material is dispersed underneath the epidermis 31 the conductive ink could be tattooed into the subject's skin 21. This may ensure that the permanent conductive contact 35 cannot be removed from the subject's skin 21.

In the example of Fig. 3 the permanent conductive contact 35 comprises a conductive ink. The conductive ink that is used for the permanent conductive contact 35 may be the same as the conductive ink that is used for the semi-permanent conductive contact 33. The conductive ink may comprise any conductive material such as silver, gold or any other suitable, non-toxic, material. In some examples the conductive material may be magnetic so as to enable the permanent conductive contact 35 to be attracted to a magnetic portion 5 of an electrode 3.

In some examples the permanent conductive contact 35 may be magnetized. In such examples the conductive ink may comprise magnetic particles which may be dispersed within the conductive ink. The magnetic particles may comprise ferromagnetic particles such as iron oxide or any other suitable materials.

Having the permanent conductive contact 35 provided on the subject's skin 21 may provide for an improved electrical connection between the subject's skin 21 and the permanent conductive contact 35 compared to the examples of Fig. 2.

The permanent conductive contact 35 may be useful for circumstances where a subject requires ongoing monitoring. For example, a subject with a chronic medical condition may require continuous monitoring of a bio-parameter and/or may require a biopotential to be measured regularly over several months or years.

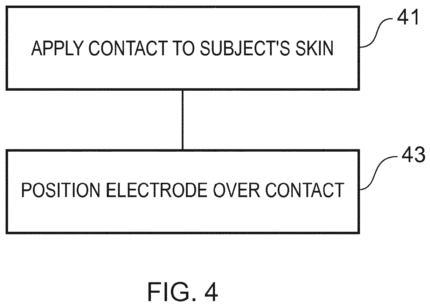

Fig. 4 illustrates an example method according to examples of the disclosure. The method could be implemented using any of the apparatus 1 and their variations described above.

The method comprises, at block 41, applying a conductive contact 23 to a subject's skin 21.

The conductive contact 23, 33, 35 may be applied to the subject's skin 21 using any suitable means. The means that are used to apply the conductive contact 23, 33, 35 to the subject's skin 21 may depend on the type of conductive contact 23, 33, 35 that is being used and the whether or not the conductive contact 23, 33, 35 is to be temporarily or permanently attached.

In examples where the conductive contact 23 is attached to the subject's skin 21 by an adhesive 25 the adhesive 25 and the conductive contact 23 may be provided on first substrate. The adhesive 25 and the conductive contact 23 may be provided on first substrate so that the adhesive 25 is provided overlaying the conductive contact 23. The adhesive 25 and the conductive contact 23 may then be transferred from the first substrate to the skin 21 of the subject. The first substrate may then be removed leaving the conductive contact 23 and the adhesive 25 applied to the subject's skin 21 with the conductive contact 23 overlaying the adhesive 25. In such examples the first substrate may be formed from a disposable material such as paper. The first substrate may be discarded once the conductive contact 23 and the adhesive 25 have been transferred.

In examples where the conductive contact 33, 35 is formed from a conductive ink the conductive contact 33, 35 may be applied to the subject's skin 21 by depositing the conductive ink on the surface of the skin 21 or by dispersing the conductive ink underneath the epidermis of the skin 21. The conductive ink may be applied in any suitable shape on the subject's skin 21. The conductive ink may be applied so as to ensure that the conductive contact 33, 35 is sized and shaped enable a good electrical connection to be formed with an electrode 3 in an apparatus 1.

The method also comprises, at block 43 positioning an electrode 3 relative to the conductive contact 23, 33, 35 to enable an electrical connection between the subject's skin 21 and the electrode 3. In some exampled the electrode 3 maybe positioned overlaying the conductive contact 23, 33, 35. In some examples positioning an electrode 3 to enable the electrical connection 35 may comprise the subject wearing at item of clothing so that the electrodes 3 within the clothing and the conductive contacts 23, 33, 35 positioned on the subject's skin 21 become aligned. In other examples positioning an electrode 3 overlying the conductive contact 23, 33, 35 may comprise a strap or other flexible substrate being attached to the subject's body.

The electrical connection that is established at block 43 enables a bio-parameter of the subject to be detected. At least one of the conductive contact 23, 33, 35 and the electrode 3 are magnetised so as to enable a magnetic field from the at least one magnetised conductive contact 23, 33, 35 and/or electrode 3 to align the conductive contact 23, 33, 35 and the electrode 3. The bio-parameter could comprise a biopotential, a biomechanical output, an optical output or any other suitable output.

In some examples the conductive contacts 23, 33, 35 and the electrodes 3 may be arranged in a magnetic pattern. This ensures that the correct electrode 3 is positioned overlaying the correct corresponding conductive contact 23, 33, 35. The magnetic pattern may comprise arranging different conductive contacts 23, 33, 35 and electrodes 3 to have different magnetic polarities so that if the apparatus 1 is arranged in an incorrect orientation there is a magnetic repulsion between one or more of the electrodes 3 and the conductive contacts 23, 33, 35.

The magnetic pattern may also be useful where a plurality of conductive contacts 23, 33, 35 are provided in proximity on the subject's skin 21. In such examples an apparatus 1 comprising a plurality of electrode 3 may be brought into proximity with the conductive contacts 23, 33, 35 on the subject's skin 21. The polarities of the magnetised conductive contacts 23, 33, 35 act to attract the corresponding electrodes 3 in the apparatus 1 so that the apparatus 1 may be automatically aligned by the magnetic forces.

Examples of the disclosure provide the advantage that when the apparatus 1 is in use the magnetic field helps to hold the electrode 3 in position relative to the conductive contacts 23, 33, 35. This helps to maintain a good electrical connection between the electrodes 3 and the conductive contacts 23, 33, 35 and so may enable reliable measurements of the bio-paremeter to be made.

Also in examples of the disclosure the magnetic fields are used to hold the apparatus 1 in place relative to the conductive contacts 23, 33, 35. This may remove the need for tightly tension straps and/or may enable a wearable electronic device to be more comfortable for a subject to wear. This may be particularly beneficial where the biopotentials are to be measured over an extended period of time.

In some examples the magnetic fields may act to return the electrodes 3 to a position overlaying the conductive contacts 23, 33, 35 if the electrodes 3 are displaced. For instance, as the apparatus 1 is being used the electrodes 3 could be accidently displaced from their correct position overlaying the conductive contacts 23, 33, 35. In such examples the magnetic field may act as a restoring force to return the electrode 3 to its original position. This may ensure that reliable measurements of the biopotential are obtained even if a user is moving.

The term "comprise" is used in this document with an inclusive not an exclusive meaning. That is any reference to X comprising Y indicates that X may comprise only one Y or may comprise more than one Y. If it is intended to use "comprise" with an exclusive meaning then it will be made clear in the context by referring to "comprising only one..." or by using "consisting".

In this brief description, reference has been made to various examples. The description of features or functions in relation to an example indicates that those features or functions are present in that example. The use of the term "example" or "for example" or "may"' in the text denotes, whether explicitly stated or not, that such features or functions are present in at least the described example, whether described as an example or not, and that they can be, but are not necessarily, present in some of or all other examples. Thus "example", "for example" or "may" refers to a particular instance in a class of examples. A property of the instance can be a property of only that instance or a property of the class or a property of a subclass of the class that includes some but not all of the instances in the class. It is therefore implicitly disclosed that a feature described with reference to one example but not with reference to another example, can where possible be used in that other example but does not necessarily have to be used in that other example.

Although embodiments of the present invention have been described in the preceding paragraphs with reference to various examples, it should be appreciated that modifications to the examples given can be made without departing from the scope of the invention as claimed.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

## Claims

1. A method comprising:
applying a conductive contact (23, 33, 35) to a subject's skin (21) ;
positioning an electrode (3) relative to the conductive contact (23, 33, 35) to enable an electrical connection between the subject's skin (21) and the electrode (3) wherein the electrical connection enables a bio-parameter of the subject to be detected; and
wherein at least one of the conductive contact (23, 33, 35) and the electrode (3) are magnetised so as to enable a magnetic field from the magnetised at least one conductive contact (23, 33, 35) and/or electrode (3) to align the conductive contact (23, 33, 35) and the electrode (3), **characterized in that** the conductive contact comprises an ink comprising magnetic particles.

2. A method as claimed in any preceding claim wherein both the conductive contact and the electrode are magnetised.

3. A method as claimed in any preceding claim wherein the electrode is provided within a wearable electronic device.

4. A method as claimed in any preceding claim wherein the electrode comprises a conductive film mounted on a textile substrate.

5. A method as claimed in any preceding claim wherein the magnetic field is arranged to hold the electrode in position overlaying the conductive contact.

6. A method as claimed in any preceding claim wherein the magnetic field is arranged to pull the electrode back to a position overlaying the conductive contact if the electrode is moved relative to the conductive portion.

7. A method as claimed in any preceding claim wherein the conductive contact comprises an adhesive and a conductive film overlaying the adhesive wherein the adhesive is applied to the surface of the subject's skin and wherein the adhesive comprises magnetic particles suspended within the adhesive.

8. A method as claimed any of claims 1 to 6 wherein the conductive contact is deposited on the surface of the subject's skin.

9. A method as claimed in any of claims 1 to 6 wherein the conductive contact comprises an ink comprising conductive particles which is dispersed underneath the epidermis of the subject's skin.

10. A method as claimed in any preceding claim wherein the electrical connection is a galvanic connection.

11. A method as claimed in any of claims 1 to 9 wherein the electrical connection comprises capacitive coupling between the conductive contact and the electrode.

12. A method as claimed in any preceding claim wherein the bio-parameter comprises at least one of; a biopotential, a bio-mechanical output, an optical output.

13. A system (1) comprising:
at least one electrode (3); and
at least one conductive contact, configured to be applied to a subject's skin,
wherein the at least one electrode (3) is arranged to be coupled to the at least one conductive contact (23, 33, 35), so as to enable an electrical connection between the conductive contact (23, 33, 35) and the electrode (3) wherein the electrical connection enables a bio-parameter of the subject to be detected;
wherein at least one of the conductive contact (23, 33, 35) and the electrode (3) are magnetised so as to enable a magnetic field to align the conductive contact (23, 33, 35) and the electrode (3), **characterized in that** the conductive contact comprises an ink comprising magnetic particles.

14. A system as claimed in claim 13 wherein the electrode is magnetised.

15. A system as claimed in claim 14 wherein the electrode comprises a magnetic portion.

## Patentansprüche

1. Verfahren, das Folgendes umfasst:
Aufbringen eines leitfähigen Kontakts (23, 33, 35) auf die Haut (21) einer Person;
Positionieren einer Elektrode (3) relativ zum leitfähigen Kontakt (23, 33, 35), um eine elektrische Verbindung zwischen der Haut (21) der Person und der Elektrode (3) zu ermöglichen, wobei die elektrische Verbindung das Detektieren eines Bioparameters der Person ermöglicht; und
wobei mindestens eines des leitfähigen Kontakts (23, 33, 35) und der Elektrode (3) magnetisiert ist, um es einem Magnetfeld des magnetisierten mindestens einen des leitfähigen Kontakts (23, 33, 35) und/oder der Elektrode (3) zu ermöglichen, den leitfähigen Kontakt (23, 33, 35) und die Elektrode (3) auszurichten, **dadurch gekennzeichnet, dass** der leitfähige Kontakt eine Tinte umfasst, die magnetische Partikel umfasst.

2. Verfahren nach einem der vorhergehenden Ansprüche, wobei sowohl der leitfähige Kontakt als auch die Elektrode magnetisiert sind.

3. Verfahren nach einem der vorhergehenden Ansprüche, wobei die Elektrode mit einer tragbaren elektronischen Vorrichtung versehen ist.

4. Verfahren nach einem der vorhergehenden Ansprüche, wobei die Elektrode eine leitfähigen Film umfasst, der an einem Textilsubstrat montiert ist.

5. Verfahren nach einem der vorhergehenden Ansprüche, wobei das Magnetfeld angeordnet ist, die Elektrode in Position zu halten, die den leitfähigen Kontakt überlagert.

6. Verfahren nach einem der vorhergehenden Ansprüche, wobei das Magnetfeld angeordnet ist, die Elektrode zurück zu einer Position zu ziehen, die den leitfähigen Kontakt überlagert, wenn die Elektrode relativ zum leitfähigen Abschnitt bewegt wird.

7. Verfahren nach einem der vorhergehenden Ansprüche, wobei der leitfähige Kontakt einen Kleber und einen leitfähigen Film, der den Kleber überlagert, umfasst, wobei der Kleber auf die Fläche der Haut der Person aufgebracht wird und wobei der Kleber magnetische Partikel umfasst, die im Kleber suspendiert sind.

8. Verfahren nach einem der Ansprüche 1 bis 6, wobei der leitfähige Kontakt auf der Fläche der Haut der Person aufgetragen ist.

9. Verfahren nach einem der Ansprüche 1 bis 6, wobei der leitfähige Kontakt eine Tinte umfasst, die leitfähige Partikel umfasst, die unter der Epidermis der Haut der Person verteilt sind.

10. Verfahren nach einem der vorhergehenden Ansprüche, wobei die elektrische Verbindung eine galvanische Verbindung ist.

11. Verfahren nach einem der Ansprüche 1 bis 9, wobei die elektrische Verbindung eine kapazitive Kopplung zwischen dem leitfähigen Kontakt und der Elektrode umfasst.

12. Verfahren nach einem der vorhergehenden Ansprüche, wobei der Bioparameter mindestens eines von Folgendem umfasst: einem Biopotenzial, einer biomechanischen Ausgabe, einer optischen Ausgabe.

13. System (1), das Folgendes umfasst:
mindestens eine Elektrode (3); und
mindestens einen leitfähigen Kontakt, der dazu ausgelegt ist, auf die Haut einer Person aufgebracht zu werden,
wobei die mindestens eine Elektrode (3) angeordnet ist, an den mindestens einen leitfähigen Kontakt (23, 33, 35) gekoppelt zu werden, um eine elektrische Verbindung zwischen dem leitfähigen Kontakt (23, 33, 35) und der Elektrode (3) zu ermöglichen, wobei die elektrische Verbindung das Detektieren eines Bioparameters der Person ermöglicht;
wobei mindestens eines des leitfähigen Kontakts (23, 33, 35) und der Elektrode (3) magnetisiert ist, um es einem Magnetfeld zu ermöglichen, den leitfähigen Kontakt (23, 33, 35) und die Elektrode (3) auszurichten, **dadurch gekennzeichnet, dass** der leitfähige Kontakt eine Tinte umfasst, die magnetische Partikel umfasst.

14. System nach Anspruch 13, wobei die Elektrode magnetisiert ist.

15. System nach Anspruch 14, wobei die Elektrode einen magnetischen Abschnitt umfasst.

## Revendications

1. Procédé comprenant :
l'application d'un contact conducteur (23, 33, 35) sur la peau (21) d'un sujet ;
le positionnement d'une électrode (3) par rapport au contact conducteur (23, 33, 35) pour permettre une connexion électrique entre la peau (21) du sujet et l'électrode (3), dans lequel la connexion électrique permet de détecter un bio-paramètre du sujet ; et
dans lequel au moins un parmi le contact conducteur (23, 33, 35) et l'électrode (3) est magnétisé de manière à permettre à un champ magnétique de l'au moins un contact conducteur (23, 33, 35) et/ou de l'électrode (3) magnétisé(e) d'aligner le contact conducteur (23, 33, 35) et l'électrode (3), **caractérisé en ce que** le contact conducteur comprend une encre comprenant des particules magnétiques.

2. Procédé selon l'une des revendications précédentes, dans lequel le contact conducteur et l'électrode sont tous deux magnétisés.

3. Procédé selon l'une des revendications précédentes, dans lequel l'électrode est prévue dans un dispositif électronique à porter.

4. Procédé selon l'une des revendications précédentes, dans lequel l'électrode comprend un film conducteur monté sur un substrat textile.

5. Procédé selon l'une des revendications précédentes, dans lequel le champ magnétique est agencé pour maintenir l'électrode en position superposée au contact conducteur.

6. Procédé selon l'une des revendications précédentes, dans lequel le champ magnétique est agencé pour ramener l'électrode en position superposée au contact conducteur si l'électrode est déplacée par rapport à la partie conductrice.

7. Procédé selon l'une des revendications précédentes, dans lequel le contact conducteur comprend un adhésif et un film conducteur superposé à l'adhésif, dans lequel l'adhésif est appliqué sur la surface de la peau du sujet, et dans lequel l'adhésif comprend des particules magnétiques en suspension dans l'adhésif.

8. Procédé selon l'une des revendications 1 à 6, dans lequel le contact conducteur est déposé sur la surface de la peau du sujet.

9. Procédé selon l'une des revendications 1 à 6, dans lequel le contact conducteur comprend une encre comprenant des particules conductrices, qui est dispersée sous l'épiderme de la peau du sujet.

10. Procédé selon l'une des revendications précédentes, dans lequel la connexion électrique est une connexion galvanique.

11. Procédé selon l'une des revendications 1 à 9, dans lequel la connexion électrique comprend un couplage capacitif entre le contact conducteur et l'électrode.

12. Procédé selon l'une des revendications précédentes, dans lequel le bio-paramètre comprend au moins un parmi : un biopotentiel, une sortie biomécanique, une sortie optique.

13. Système (1) comprenant :
au moins une électrode (3) ; et
au moins un contact conducteur configuré pour être appliqué sur la peau d'un sujet,
dans lequel l'au moins une électrode (3) est agencé pour être couplée à l'au moins un contact conducteur (23, 33, 35), de manière à permettre une connexion électrique entre le contact conducteur (23, 33, 35) et l'électrode (3), dans lequel la connexion électrique permet de détecter un bio-paramètre du sujet ;
dans lequel au moins un parmi le contact conducteur (23, 33, 35) et l'électrode (3) est magnétisé de manière à permettre à un champ magnétique d'aligner le contact conducteur (23, 33, 35) et l'électrode (3), **caractérisé en ce que** le contact conducteur comprend une encre comprenant des particules magnétiques.

14. Système selon la revendication 13, dans lequel l'électrode est magnétisée.

15. Système selon la revendication 14, dans lequel l'électrode comprend une partie magnétique.
